# EUROPÄISCHE PATENTSCHRIFT

(11) **EP 1 221 903 B1**
(45) Veröffentlichungstag und Bekanntmachung des Hinweises auf die Patenterteilung: **28.06.2006**
(21) Anmeldenummer: 01971871.7
(22) Anmeldetag: 04.08.2001
(51) Int. Cl.: A61B 18/14

(54) **UROLOGISCHES RESEKTOSKOP MIT EINER MONOPOLAREN ODER BIPOLAREN ELEKTRODE**
UROLOGICAL RESECTOSCOPE WITH A MONOPOLAR OR BIPOLAR ELECTRODE
RESECTOSCOPE UROLOGIQUE AVEC UNE ELECTRODE MONOPOLAIRE OU BIPOLAIRE

(30) Priorität: 26.08.2000 DE 10042096; 26.04.2001 DE 20107176 U
(43) Veröffentlichungstag der Anmeldung: 17.07.2002
(73) Patentinhaber: OLYMPUS WINTER & IBE GMBH, 22045 Hamburg (DE)
(72) Erfinder: BROMMERSMA, Pieter, 22941 Bargteheide (DE); NUSSBAUM, Felix, 22089 Hamburg (DE)
(74) Vertreter: Schaefer, Konrad
(86) Internationale Anmeldenummer: PCT/EP2001/009044
(87) Internationale Veröffentlichungsnummer: WO 2002/017807

(56) Entgegenhaltungen:
- DE-A- 3 917 583
- DE-A- 3 918 316
- FR-A- 2 400 351

## Beschreibung

Die Erfindung betrifft ein urologiscbes Resektoskop der im Oberbegriff des Anspruch 1 genannten Art.

Gattungsgemäße Resektoskope sind in erster Linie für die Prostataresektion vorgesehen, jedoch ihrer Bauart nach gegebenenfalls auch für andere chirurgische Einsätze verwendbar. Unter Resektoskop werden hier endoskopische Geräte verstanden, bei denen in einem Schaftrohr eine Optik und ein monopolarer oder bipolarer Elektrodenträger mit ein oder zwei distalen Elektroden, z.B. eine Resektions- und eine Neutralelektrode, angeordnet sind, wobei der Elektrodenträger mit den Elektroden in Achsrichtung verschiebbar gelagert ist und mit seinem proximalen Ende an einem Schiebekörper des Resektoskopes befestigt und elektrisch kontaktiert ist, welcher mit einer Griftbetätigung von Hand axial verstellt werden kann, um die Elektrode axial zu verschieben.

Bei der Prostataresektion wird das Resektoskop mit dem distalen Ende des Schaftrohres durch die Harnröhre bis in das Innere der Prostata vorgeschoben.

Bei eingeschalteter HF-Beaufschlagung der Elektrode kann diese durch Handbewegung des Schiebekörpers, an dem sie proximal befestigt ist, vor- und zurückgeschoben werden, um Gewebe zu schneiden. Die Elektrode weist dazu in üblicher Weise distal eine als Drahtschlinge ausgebildete Resektionselektrode auf, mit der Gewebeschnipsel resiziert werden können. Die Elektrode kann auch anders ausgebildet sein, beispielsweise als Knopfelektrode, Ivlessere1ektrode, Rollenelektrode oder dergleichen, um für unterschiedliche Einsatzzwecke verwendet werden zu können, z. B. zum Koagulieren, Schneiden und dergleichen.

Die klassische Resektoskopie bedient sich der monopolaren bzw. unipolaren Technik. Dabei fließt ein HF-Strom von der Resektionselektrode, der aktiven Elektrode, durch den Körper des Patienten zu einer großflächigen Neutralelektrode, die außen am Patienten, beispielsweise an dessen Oberschenkel, angeordnet ist.

Mit dem Stromfluß durch den Körper des Patienten sind aber Risiken verbunden, die selbst bei sachgemäßer Handhabung des Reseh-toskopes niemals gänzlich auszuschalten sind. Zu nennen ist in diesem Zusammenhang insbesondere das Auftreten von unkontrollierbaren Leckströmen bzw. vagabundierenden Strömen, die z.B. bei Kontakt des Patienten mit einem metallischen Gegenstand, wie etwa dem Operationstisch, zu schmerzhaften Hautverbrennungen führen können. Weiterhin besteht bei strominduzierten Muskelkontraktionen die Gefahr, daß der Patient unkontrollierte, plötzliche Bewegungen ausführt, die zu Schnittverletzungen durch das Resektoskop führen können. Es besteht zudem latent die Gefahr, daß Muskel oder Nerven in der Umgebung des Resektionsgebietes zumindest zeitweise durch vagabundierende Ströme geschädigt werden.

Die oben genannten Risiken lassen sich bei Anwendung bipolarer Techniken weitestgehend ausschalten. Diesen ist gemein, daß nicht nur die Aktivelektrode, sondern auch die Neutral- bzw. Returnelektrode in den Körper des Patienten eingebracht wird, so daß der HF-Strom nur zwischen den beiden Elektroden am Elektrodenträger fließt, nicht aber, oder nur über definierte kurze Strecken, durch den Körper des Patienten. Eine solche bipolare Elektrode zeigt die DE-OS-25 21 719, bei der also der Elektrodenträger von zwei Leiterdrähten durchlaufen wird.

Alternativ ist es bei bipolaren Elektroden auch bekannt, statt der Neutralelektrode eine zweite aktive Elektrode als Returnelektrode vorzusehen, wobei der Stromfluß zwischen diesen beiden in Gewebekontakt stehenden Elektroden erfolgt. Eine solche bipolare Elektrode zeigt die WO 96/23449.

Ein Problem sowohl der monopolaren wie auch der bipolaren Elektroden bildet dabei stets die ordnungsgemäße Kontaktierung der die Elektroden mit Strom versorgenden Leiterdrähte an ihren Kontaktabschnitten im Schiebekörper. Dort muß der Kontakt mit weiterführenden Kabeln hergestellt werden, die zu den Polen eines getrennt aufgestellten HF-Generators führen. Kontaktstellen mit HF-Beaufschlagung sind problematisch und neigen zu Kontaktfehlern bzw. zum Verschmoren.

Bei älteren Konstruktionen mit monopolarer Technik wurde im Schiebekörper mit einer Klemmschraube gleichzeitig kontaktiert und die mechanische Befestigung des Elektrodenträgers sichergestellt. Verschmort diese Kontaktierungsstelle, so muß der gesamte Schiebekörper ausgewechselt werden.

Eine gattungsgemäße, monopolare Konstruktion ist aus der US 4,917,621 und der US 4,919,131 jeweils in Fig. 3 bekannt. Der Schiebekörper weist einen quer durchgehenden Schacht auf, in den der Stecker des weiterführenden HF-Kabels zur Kontaktierung des im Schacht frei liegenden Kontaktabschnitts des Elektrodenträgers einsteckbar ist. Distal vom Schacht ist eine an einem Befestigungsabschnitt des Elektrodenträgers angreifende Klemmeinrichtung vorgesehen.

Vorteilhaft an dieser Konstruktion ist die Möglichkeit, den Elektrodenträger mit der Klemmeinrichtung am Schiebekörper gesondert mechanisch zu befestigen, so daß seine ordnungsgemäße mechanische Funktion zunächst ausprobiert werden kann. Anschließend kann mit dem Stecker kontaktiert werden. Verschmort die Kontaktierungsstelle, so muß nur der Elektrodenträger und das Kabel mit dem Stecker gewechselt werden. Die Klemmeinrichtung und der Schiebekörper bleiben unversehrt, da die Klemmeinrichtung gesondert ausgebildet ist.

Diese bekannte Konstruktion weist jedoch auch Nachteile auf. Da die Befestigungseinrichtung distal von der Kontaktiereinrichtung liegt, wird die Stelle des Elektrodenträgers, an der die Befestigung erfolgt, von dem Leiter durchlaufen, der den Kontaktabschnitt mit der Aktivelektrode verbindet. Folglich ist der Elektrodenträger an dieser Stelle nicht sehr stabil. Die Befestigungseinrichtung muß darauf Rücksicht nehmen und kann beispielsweise nur mit sehr geringen Klemmkräften arbeiten. Erfolgt die Befestigung mit einem in eine Nut auf dem Elektrodenträger eingreifenden Schieber, so kann die Nut nur sehr flach ausgebildet sein, woraus sich eine verringerte Befestigungssicherheit ergibt.

Der proximale Endbereich des Elektrodenträgers wird von dem Bereich gebildet, an dem die Befestigung erfolgt, sowie von dem Kontaktierbereich. Diese Bereiche, also der gesamte Endbereich des Elektrodenträgers, sind folglich starr und biegesteifer als der restliche Teil des Elektrodenträgers, der nur aus einem Innenleiter und einer Außenisolierung besteht. Bei Resektoskopen liegt jedoch üblicherweise der Elektrodenträger im Schaftrohr dicht benachbart neben der Optik, während er im Bereich des Schiebekörpers im größeren Abstand zu diesem liegen muß, um Platz zu schaffen für die Kontaktiereinrichtung und die Befestigungseinrichtung. Folglich muß der Elektrodenträger im Hauptkörper verschwenkt geführt werden, wie dies z. B. in der WO 96/23449, Fig. 13 angedeutet ist. Da wiederum der Hauptkörper aus konstruktiven Gründen nicht zu lang sein darf, muß auf kurzem Wege stark verschwenkt werden. Hierbei stört jedoch die erhebliche Länge des starreren Endbereiches des Elektrodenträgers der bekannten Konstruktion.

Ferner sind Fehlmontagen möglich, wenn der Elektrodenträger nicht weit genug eingesteckt und dann befestigt und kontaktiert wird.

Bei der gattungsgemäßen WO 96/23449, Fig. 16, die eine bipolare Elektrode zeigt, weist der Elektrodenträger im Bereich des Schiebekörpers zwei Kontaktabschnitte auf. Ein auf den Schiebekörper aufsteckbarer Stecker des weiterführenden Kabels kontaktiert beide Kontaktabschnitte, wobei er an beiden Kontaktabschnitten sowohl kontaktiert als auch klemmt. Eine besondere, getrennte Befestigungseinrichtung ist nicht vorgesehen. Eine Befestigung des Elektrodenträgers zum Ausprobieren der mechanischen Funktion vor dem Kontaktiervorgang ist nicht möglich.

Die Aufgabe der vorliegenden Erfindung besteht darin, ein verbessertes gattungsgemäßes urologisches Resektoskop monopolarer oder bipolarer Bauart zu schaffen, bei der der Elektrodenträger störungsfrei im Schiebekörper befestigt und kontaktiert werden kann.

Diese Aufgabe wird mit den Merkmalen des Anspruches 1 gelöst.

Erfindungsgemäß ist am Elektrodenträger der Elektrode der Befestigungsabschnitt getrennt und proximal vom ersten Kontaktabschnitt vorgesehen. Entsprechend ist bei dem Schiebekörper des Resektoskopes die Befestigungseinrichtung proximal von der ersten Kontaktiereinrichtung vorzusehen.

Durch die getrennte Ausbildung ist der Elektrodenträger zum einen mit der Klemmeinrichtung am Schiebekörper gesondert mechanisch zu befestigen, so daß seine ordnungsgemäße mechanische Funktion zunächst ausprobiert werden kann. Anschließend kann mit einem Stecker kontaktiert werden. Verschmort die Kontaktierungsstelle, so muß nur der Elektrodenträger und das Kabel mit dem Stecker gewechselt werden. Die Klemmeinrichtung und der Schiebekörper bleiben unversehrt, da die Klemmeinrichtung gesondert ausgebildet ist. Bei dieser Ausbildung der Elektrode ergibt sich weiterhin der Vorteil, daß der Befestigungsabschnitt des Elektrodenträgers nicht mehr von einem Innenleiter durchlaufen wird und daher sehr stabil ausgebildet sein kann. Es können verschiedene sehr sicher wirkende Befestigungsmethoden verwendet werden, z.B. Klemmung mit hoher Klemmkraft, Verriegelung auf tiefen Nuten oder sogar Verriegelung mit einem Stift durch eine Querbohrung des Elektrodenträgers. Es lassen sich auch gut haltende Rastverbindungen vorsehen. Ein weiterer Vorteil der proximalen Anordnung des Befestigungsabschnittes besteht darin, daß bei ordnungsgemäßer Befestigung sichergestellt ist, daß der Elektrodenträger vollständig eingesteckt ist, also an der Stelle der Kontaktiereinrichtung der Kontaktabschnitt des Elektrodenträgers liegt und somit eine Kontaktierung erfolgen kann. Schließlich läßt sich auch sehr wirkungsvoll das Problem der Verschwenkung des steifen Endabschnittes des Elektrodenträgers beim Einführen durch den Hauptkörper lösen. Das biegesteife Endstück bestehend aus dem Kontaktabschnitt und dem Befestigungsabschnitt läßt sich kürzer ausbilden. Der Befestigungsabschnitt des Elektrodenträgers kann ferner auch mit geringerem Durchmesser ausgebildet werden, was ebenfalls die Durchführung durch eine engere Verschwenkung ermöglicht.

Nach Anspruch 2 wird für eine bipolare Elektrode mit Vorteil vorgeschlagen, daß der Befestigungsabschnitt getrennt von beiden Kontaktabschnitten und proximal von zumindest einem der Kontaktabschnitte angeordnet ist. So kann zunächst seine ordnungsgemäße mechanische Funktion ausprobiert werden, bevor mit einem Stecker kontaktiert wird.

Es ergibt sich für eine Elektrode gemäß den Merkmalen des Anspruch 2 zum einen die Möglichkeit, den ersten Kontaktabschnitt proximal und den zweiten distal vom Befestigungsabschnitt anzuordnen. Entsprechend wäre die Befestigungseinrichtung zwischen den beiden Kontaktiereinrichtungen im Schiebekörper anzuordnen. Es würde in vorteilhafter Weise nur ein Innenleiter den Befestigungsabschnitt durchlaufen, wodurch mit höherer Klemmkraft auf dem Befestigungsabschnitt geklemmt werden kann. Weiterhin würde dies die Isolierung der beiden Kontaktabschnitte erleichtern, da sie bereits durch den Befestigungsabschnitt getrennt werden, soweit dieser aus einem isolierenden Material hergestellt ist, z.B. einer nicht-leitenden Keramik.

Mit Vorteil werden allerdings die Merkmale des Anspruch 3 vorgeschlagen. Danach sind beide Kontaktabschnitte distal vom Befestigungsabschnitt anzuordnen. Dann ist bei dem Schiebekörper des Resektoskopes die Befestigungseinrichtung auch proximal von den beiden Kontaktiereinrichtungen vorzusehen. Bei dieser Ausbildung der Elektrode ergibt sich zunächst der Vorteil, daß der Befestigungsabschnitt des Elektrodenträgers von keinem der Innenleiter durchlaufen wird und daher sehr stabil ausgebildet sein kann. Es ergeben sich die Vorteile bei der Befestigung, die bereits beim Anspruch 1 am Beispiel einer monopolaren Elektrode erläutert wurden. Ein weiterer Vorteil der proximalen Anordnung des Befestigungsabschnittes besteht darin, daß bei ordnungsgemäßer Befestigung sichergestellt ist, daß der Elektrodenträger vollständig eingesteckt ist, daß also an den Stellen der Kontaktiereinrichtungen auch die Kontaktabschnitte des Elektrodenträgers liegen und somit eine fehlerlose Kontaktierung erfolgen kann. Schließlich läßt sich auch hier sehr wirkungsvoll das Problem der Verschwenkung des steifen Endabschnittes des Elektrodenträgers beim Einführen durch den Hauptkörper lösen. Das biegesteife Endstück bestehend aus den beiden Kontaktabschnitten und dem Befestigungsabschnitt läßt sich kurz ausbilden, wobei die Isolierung zwischen den Kontaktabschnitten gewährleistet bleiben muß. Der Befestigungsabschnitt des Elektrodenträgers kann ferner auch mit geringerem Durchmesser ausgebildet werden, was ebenfalls die Durchführung durch eine engere Verschwenkung verbessert.

Vorteilhaft sind die Merkmale des Anspruch 4 vorgesehen. Diese Ausbildung wird erst durch die Erfindung ermöglicht, denn erst sie gestattet es, den Befestigungsabschnitt völlig getrennt von der übrigen Konstruktion des Elektrodenträgers in Richtung auf gute Befestigbarkeit auszulegen. Dabei kann vorteilhaft gemäß Anspruch 5 eine durchgehend massive Metallkonstruktion gewählt werden.

Gemäß Anspruch 6 können vorteilhaft der Befestigungsabschnitt und wenigstens ein Teil eines benachbarten Kontaktierabschnittes einstückig ausgebildet sein. Dadurch läßt sich die Konstruktion insbesondere hinsichtlich ihrer Herstellbarkeit vereinfachen und erhält höhere Festigkeit. Wenn beide Kontaktierabschnitte distal vom Befestigungsabschnitt angeordnet sind, ist erfindungsgemäß zumindest der proximale Teil des zum Befestigungsabschnitt benachbarten Kontaktabschnittes einstückig mit dem Befestigungsabschnitt auszubilden. Wenn der Befestigungsabschnitt zwischen den beiden Kontaktabschnitten ausgebildet ist, so ist wahlweise entweder zumindest der distale Teil des proximal zum Befestigungsabschnitt benachbarten Kontaktabschnittes einstückig mit dem Befestigungsabschnitt auszubilden, oder zumindest der proximale Teil des distal zum Befestigungsabschnitt benachbarten Kontaktabschnittes einstückig mit dem Befestigungsabschnitt auszubilden.

Nach Anspruch 7 wird mit Vorteil vorgeschlagen, den Befestigungsabschnitt aus isolierendem Material herzustellen, wenn er zwischen den beiden Kontaktabschnitten angeordnet ist. Er trägt dann bereits zur Isolierstrecke bei, und erlaubt so eine insgesamt kürzere Ausbildung des proximalen Endbereichs der Elektrode.

In den Zeichnungen ist die Erfindung beispielsweise und schematisch dargestellt.
Es zeigen:
- Figur 1: einen Achsschnitt eines Resektoskopes mit einem ersten Ausführungsbeispiel einer montierten monopolaren Elektrode, und
- Figur 2: einen Achsschnitt des proximalen Endbereiches eines Resektoskopes mit einem ersten Ausführungsbeispiel einer montierten bipolaren Elektrode, und
- Figur 3: einen Achsschnitt des proximalen Endbereiches eines Resektoskopes mit einem zweiten Ausführungsbeispiel einer montierten bipolarer Elektrode.

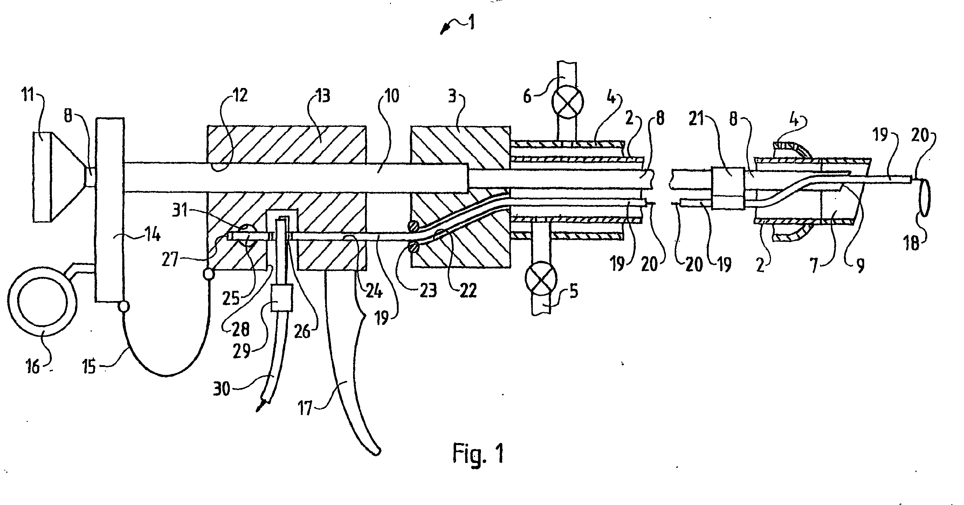

Das in Figur 1 dargestellte Resektoskop 1 weist ein Schaftrohr 2 auf, das mit seinem proximalen Ende an einem Hauptkörper 3 befestigt ist. In nicht dargestellter Weise kann das Schaftrohr 2 mit einer üblichen Kupplungseinrichtung abnehmbar am Hauptkörper 3 befestigt sein. Um das Schaftrohr 2 herum ist ein Außenrohr 4 vorgesehen, das ebenfalls am Hauptkörper 3 befestigt ist und zwar ebenfalls in der üblichen Weise mit einer nicht dargestellten Kupplung. Das Innere des Schaftrohres 2 dient in üblicher Weise als Zulaufkanal für die Dauerspülung und ist, wie Fig. 1 zeigt, von außen über einen mit Ventil versehenen Anschluß 5 erreichbar, an den ein Schlauch anschließbar ist. Ein weiterer gleichartiger Anschluß 6 zum Anschließen eines weiteren Schlauches ist an den Ringspalt zwischen Schaftrohr 2 und Außenrohr 4 angeschlossen, der als Rücklaufkanal dient.

Die beiden Rohre 2, 4 bestehen in üblicher Ausbildung aus Metall. Der distale Endabschnitt des Schaftrohres 2 ist in üblicher Weise isolierend ausgebildet, beispielsweise als Keramikendstück 7.

Im Inneren des Schaftrohres 2 verläuft achsparallel eine Optik 8, die in der dargestellten Montagestellung mit ihrem distalen Objektiv 9 den Arbeitsbereich vor dem Keramikendstück 7 betrachtet und die proximal den Hauptkörper 3 durchläuft. Sie durchläuft von dort weiter ein im Hauptkörper 3 befestigtes Führungsrohr 10 und endet jenseits von dessen proximalem Ende mit einem Okular 11, an dessen Stelle auch eine Kamera sitzen kann.

Auf dem Führungsrohr 10 ist mit einer Führungsbohrung 12 ein Schiebekörper 13 in axialer Richtung verschiebbar gelagert. Am proximalen Ende des Führungsrohres 10 ist ein Endstück 14 befestigt, demgegenüber der Schiebekörper 13 im dargestellten Ausführungsbeispiel mit der üblichen Blattfeder 15 federnd abgestützt ist. Am Endstück 14 ist ein Daumenring 16 angeordnet, während am Schiebestück 13 ein Fingergriff 17 angeordnet ist. Mit einer Hand des Operateurs kann mit dem Daumen im Daumenring 16 und mit dem Zeigefinger am Fingergriff 17 angefaßt und der Schiebekörper 13 axial bewegt werden. Alternativ kann anstelle der erläuterten "aktiven" Betätigung auch eine "passive" Betätigung vorgesehen sein, bei der die Blattfeder 15 zwischen Schiebekörper 13 und Hauptkörper 3 angeordnet ist und auch die Angriffstellen 16, 17 an diesen Teilen sitzen.

Im dargestellten Resektoskop ist auswechselbar eine HF-beaufschlagbare monopolare Elektrode 18 vorgesehen, die in üblicher Ausbildung für die Prostataresektion als rechtwinklig zur Achsrichtung erstreckte Drahtschlinge ausgebildet ist. Die Elektrode 18 wird von einem Elektrodenträger 19 getragen, der als Außenisolierung mit innerem Leiterdraht 20 ausgebildet ist. Der Elektrodenträger 19 ist in üblicher Ausbildung mit einer Hülse 21 längsverschiebbar auf der Optik 8 gelagert und durchläuft das Schaftrohr 2 bis zum Hauptkörper 3. Dort durchläuft er einen seitlich verschwenkten Durchgangskanal 22 mit Ringdichtung 23 zur Flüssigkeitsabdichtung und verläuft von dessen proximaler Mündung unter größerem Achsabstand wiederum parallel zur Achse weiter bis in eine Aufnahmebohrung 24 im Schiebekörper 13. Anstelle der Aufnahmebohrung 24 kann als alternative Ausbildung der Aufnahme für den Elektrodenträger 19 z.B. auch eine nach proximal konisch zulaufende Öffnung, ein zur Seite offener Schlitz oder dergleichen vorgesehen sein.

Im proximalen Endbereich weist der Elektrodenträger 19 einen sein Endstück ausbildenden Befestigungsabschnitt 25 auf, der ausreichend fest, z.B. massiv, aus Metall ausgebildet ist, um dort den Elektrodenträger 19 sicher mechanisch befestigen zu können. Distal anschließend weist der Elektrodenträger 19 einen Kontaktabschnitt 26 auf, der mit einer elektrisch leitenden Außenfläche versehen ist, welche mit dem Leiterdraht 20 des Elektrodenträgers 19 elektrisch leitend verbunden ist.

Die Aufnahmebohrung 24 weist ferner in Form ihres proxi-malen Endes 27 einen Endanschlag für den Elektrodenträger 19 auf, bis zu dem dieser in proximaler Richtung in die Aufnahmebohrung 24 einsteckbar ist.

Ist der Elektrodenträger 19 in der dargestellten Montagestellung bis zum Endanschlag 27 in die Aufnahmebohrung 24 des Schiebekörpers 13 eingesteckt, so liegt er mit seinem Kontaktabschnitt 26 in einer Ausnehmung 28 des Schiebekörpers 13, in dem der Kontaktabschnitt 26 von außen frei zugänglich ist. Er kann dort beispielsweise mit dem dargestellten Klemmstecker 29 am Ende eines zu einem nicht dargestellten HF-Generator weiterführenden Kabels 30 kontaktiert werden.

Unmittelbar proximal neben der Ausnehmung 28 im Bereich des Befestigungsabschnittes 25 des Elektrodenträgers 19 ist im Schiebekörper 13 eine Befestigungseinrichtung vorgesehen, die im Ausführungsbeispiel eine Querbohrung 31 aufweist, welche beispielsweise mit Innengewinde zum Einschrauben einer Klemmschraube versehen sein kann. Alternativ kann die Befestigungseinrichtung auch anders ausgebildet sein z.B. mit einem Schieber, der in eine Nut faßt, als Rastverbindung oder dergleichen.

Die Ausnehmung 28 kann, wie dargestellt, allseitig freien Zugang zu dem Kontaktabschnitt 26 des Elektrodenträgers 19 bieten oder sie kann beispielsweise auch als nur von einer Seite zugangsvermittelnder Schacht ausgebildet sein, z.B. gemäß US 4,919,131.

Ist der Elektrodenträger 19 im Schiebekörper 13 ordnungsgemäß befestigt und kontaktiert, so kann durch die beschriebene Schiebebewegung des Schiebekörpers 13 der gesamte Elektrodenträger 19 mit der Elektrode 18 gegenüber dem Schaftrohr 2 längs verschoben werden. Unter Beobachtung durch die Optik 8 kann bei Hochfrequenzbeaufschlagung der Elektroden 18 und 118 mit diesen gleichzeitig schneidend und koagulierend unter axialer Bewegung gearbeitet werden.

Zum Auswechseln des Elektrodenträgers 19 wird der Klemmstecker 29 abgenommen und die Befestigungseinrichtung (Querbohrung 31) gelöst. Dann kann der Elektrodenträger komplett aus dem Resektoskop 1 in distaler Richtung herausgezogen werden. In umgekehrter Weise kann eine neue Elektrode in proximaler Richtung bis zum Endanschlag 27 eingeschoben, mechanisch befestigt und kontaktiert werden. Dabei ist es möglich, zunächst den Elektrodenträger 19 an der Querbohrung 31 mechanisch zu befestigen und die ordnungsgemäße Funktion durch Hin- und Herschieben des Schiebekörpers 13 auszuprobieren, bevor die Kontaktierung mit dem Klemmstecker 29 erfolgt.

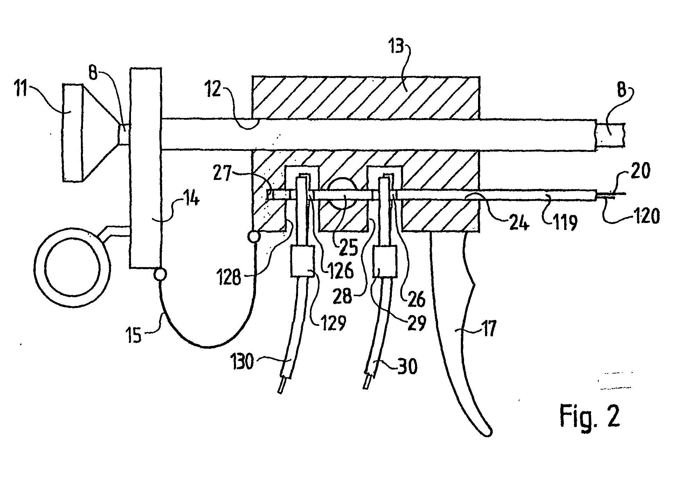

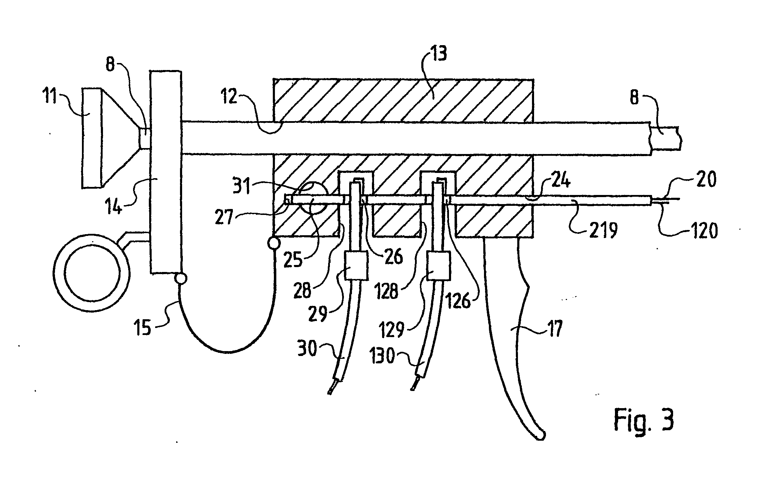

In den Figuren 2 und 3 sind zwei zueinander alternative Beispiele erfindungsgemäßer bipolarer Elektroden im montierten Zustand gezeigt, wobei für gleiche Teile gleiche Bezugszeichen wie in Figur 1 gewählt wurden. Beispiele von typischen bipolaren Elektroden zeigen die DE 100 28 850.2 sowie die darin zitierten Schriften. Deshalb wird hier auf die Darstellung des distalen Bereiches der bipolaren Elektroden in den Figuren 2 und 3 verzichtet.

Figur 2 zeigt, daß im Inneren des isolierenden Elektrodenträgers 119 zwei Leiterdrähte 20 und 120 vorgesehen sind, die in distaler Richtung bis zur Aktivelektrode bzw. bis zur Returnelektrode verlaufen, und diese leitend mit einem ersten Kontaktabschnitt 26 und einem zweiten Kontaktabschnitt 126 verbinden, die voneinander ebenso elektrisch isoliert sind, wie auch die Leiterdrähte 20 und 120, und die z. B. mit einem Doppelstecker kontaktiert werden können. In Analogie zum hier gezeigten Beispiel ist es auch möglich, die Returnelektrode leitend mit dem ersten und die Aktivelektrode leitend mit dem zweiten Kontaktabschnitt zu verbinden.

Zwischen den beiden Kontaktabschnitten 26 und 126 weist der Elektrodenträger 119 einen Befestigungsabschnitt 25 auf, der ausreichend fest ausgebildet ist, um dort den Elektrodenträger 119 sicher mechanisch befestigen zu können. Der Befestigungsabschnitt 25 kann beispielsweise aus einem isolierendem Material ausreichender Festigkeit hergestellt sein. Distal anschließend weist der Elektrodenträger 119 wie bisher einen ersten Kontaktabschnitt 26 auf, der mit einer elektrisch leitenden Außenfläche versehen ist, welche mit dem Leiterdraht 20 des Elektrodenträgers 119 elektrisch leitend verbunden ist.

Weiterhin weist der Elektrodenträger 119 proximal vom Befestigungsabschnitt 25 einen zweiten Kontaktabschnitt 126 auf, der ebenfalls mit einer elektrisch leitenden Außenfläche versehen ist, welche mit dem zweiten Leiterdraht 120 des Elektrodenträgers 119 elektrisch leitend verbunden ist.

Wie bereits in Figur 1 weist die Aufnahmebohrung 24 in Form ihres proximalen Endes 27 einen Endanschlag für den Elektrodenträger 119 auf, bis zu dem dieser in proximaler Richtung in die Aufnahmebohrung 24 einsteckbar ist.

Ist der Elektrodenträger 119 in der dargestellten Montagestellung bis zum Endanschlag 27 in die Aufnahmebohrung 24 des Schiebekörpers 13 eingesteckt, so liegt er mit seinem Kontaktabschnitt 26 in einer Ausnehmung 28 des Schiebekörpers 13, in dem der Kontaktabschnitt 26 von außen frei zugänglich ist. Gleichzeitig liegt er mit seinem Kontaktabschnitt 126 in einer zweiten Ausnehmung 128 des Schiebekörpers 13, in dem der zweite Kontaktabschnitt 126 von außen ebenfalls frei zugänglich ist. Beide können dort beispielsweise mit den dargestellten Klemmsteckern 29 und 129 mit den Enden von zu den Polen eines nicht dargestellten HF-Generators führenden Kabeln 30 und 130 kontaktiert werden. Die Kontaktierung kann auch mit einem Doppelstecker erfolgen, der gleichzeitig den Kontakt mit den beiden Kontaktabschnitten 26 und 126 herstellt.

Schließlich zeigt Figur 3 einen zweiten auswechselbaren, HF-beaufschlagbaren, bipolaren Elektrodenträger 219, der als Außenisolierung mit inneren Leiterdrähten 20 und 120 ausgebildet ist. Im proximalen Endbereich weist der Elektrodenträger 219 einen sein Endstück ausbildenden Befestigungsabschnitt 25 auf, der ausreichend fest, z.B. massiv, aus Metall ausgebildet ist, um dort den Elektrodenträger 19 sicher mechanisch befestigen zu können. Distal anschließend weist der Elektrodenträger 219 wie bisher einen ersten Kontaktabschnitt 26 auf, der mit einer elektrisch leitenden Außenfläche versehen ist, welche mit dem Leiterdraht 20 des Elektrodenträgers 219 elektrisch leitend verbunden ist. Distal vom ersten Kontaktabschnitt 26 und davon elektrisch getrennt schließt sich ein zweiter Kontaktabschnitt 126 an, der ebenfalls mit einer elektrisch leitenden Außenfläche versehen ist, welche nunmehr mit dem Leiterdraht 120 des Elektrodenträgers 219 elektrisch leitend verbunden ist.

Ist der Elektrodenträger 219 in der dargestellten Montagestellung bis zum Endanschlag 27 in die Aufnahmebohrung 24 des Schiebekörpers 13 eingesteckt, so liegt er mit seinem ersten Kontaktabschnitt 26 in einer Ausnehmung 28 des Schiebekörpers 13, in dem der Kontaktabschnitt 26 von außen frei zugänglich ist. Gleichzeitig liegt er mit seinem zweiten Kontaktabschnitt 126 in einer zweiten Ausnehmung 128 des Schiebekörpers 13, in dem der Kontaktabschnitt 126 frei zugänglich ist. Beide können in den Ausnehmungen 28, 128 beispielsweise mit den dargestellten Klemmsteckern 29 und 129 am Ende von weiterführenden Kabeln 30 und 130 kontaktiert werden, die zu den beiden Polen eines nicht dargestellten HF-Generators führen.

Unmittelbar proximal neben der ersten Ausnehmung 28 im Bereich des Befestigungsabschnittes 25 des Elektrodenträgers 219 ist im Schiebekörper 13 eine Befestigungseinrichtung vorgesehen, die der in Figur 1 bereits beschriebenen entspricht.

Die Ausnehmungen 28 und 128 können, wie dargestellt, allseitig freien Zugang zu den Kontaktabschnitten 26 und 126 des Elektrodenträgers 219 bieten oder sie können beispielsweise auch als nur von einer Seite zugangsvermittelnde Schächte ausgebildet sein, z.B. gemäß US 4,919,131.

Zum Auswechseln des Elektrodenträgers 219 werden die Klemmstecker 29 und 129 abgenommen und die Befestigungseinrichtung (Querbohrung 31) gelöst. Dann kann der Elektrodenträger 219 komplett aus dem Resektoskop 1 in distaler Richtung herausgezogen werden. In umgekehrter Weise kann eine neue Elektrode in proximaler Richtung bis zum Endanschlag 27 eingeschoben, mechanisch befestigt und kontaktiert werden. Dabei ist es möglich, zunächst den Elektrodenträger 19 an der Querbohrung 31 mechanisch zu befestigen und die ordnungsgemäße Funktion durch Hin- und Herschieben des Schiebekörpers 13 auszuprobieren, bevor die Kontaktierung mit den Klemmsteckern 29 und 129 erfolgt.
Anstelle der hier dargestellten Ausführungsbeispiele kann die Elektrode 18 auch anders geformt sein, z.B. als Knopfelektrode, Stiftelektrode, Rollenelektrode oder Elektrode in Messerform, die bei HF-Beaufschlagung koagulierend, vaporisierend oder schneidend arbeiten. Im Falle einer bipolaren Elektrode kann eine der Elektroden als Neutralelektrode ausgebildet sein, die nicht in Gewebekontakt kommt, während die andere Elektrode die Aktivelektrode bildet. Genauso können aber auch beide Elektroden in Gewebekontakt stehen, und gleichzeitig z.B. koagulierend und resizierend wirken.

In allen dargestellten Ausführungsbeispielen der Figuren 1 bis 3 ist als Endanschlag für das Einstecken des Elektrodenträgers 19 , 119 bzw. 219 jeweils das Ende 27 der Aufnahmebohrung 24 vorgesehen. Ein Endanschlag kann jedoch auch an der Klemmvorrichtung selbst vorgesehen sein, beispielsweise an der in die Gewindebohrung 31 anzuschraubenden Klemmschraube. Falls als Klemmeinrichtung beispielsweise ein Schieber vorgesehen ist, der in eine Nut auf dem Befestigungsabschnitt 25 eingreift, so kann auch an diesem Schieber ein geeigneter Anschlag vorgesehen sein, der in geeigneter Weise z.B. mit einem entsprechenden Anschlag auf dem Befestigungsabschnitt 25 zusammenwirkt.

In den Ausführungsbeispielen ist dargestellt, daß im Schiebekörper 13 der ordnungsgemäß montierte Elektrodenträger 19 , 119 oder 219 mit seinen Kontaktabschnitten 26 und 126 in Ausnehmungen 28 und 128 nach außen frei zugänglich angeordnet ist, um wie dargestellt mit von außen herangeführten Steckern 29 oder 129 kontaktiert zu werden, die auch als Doppelstecker ausgebildet sein können. Es sind aber auch andere Kontaktierungseinrichtungen möglich, bei denen bei= spielsweise der Kontaktabschnitt 26 mit einem Kontakt berührt wird, der am Schiebekörper 13, z.B. in dessen Innerem, befestigt ist und von dem ein Kabel auf andere als dargestellte Weise weiterführend verläuft. In diesem Fall kann die Ausnehmung 28 entfallen. Dies gilt in gleicher Weise für den zweiten Kontaktabschnitt 126 und die zweite Ausnehmung 128.

In den dargestellten Ausführungsbeispielen sind der Befestigungsabschnitt 25 und die Kontaktabschnitte 26 und 126 in getrennter Ausbildung dargestellt. Es kann aber auch einer der Kontaktabschnitte einstückig mit dem Befestigungsabschnitt ausgebildet sein, z.B. Kontaktabschnitt 26 in den Figuren 1, 2 und 3 oder Kontaktabschnitt 126 in der Figur 2.

Bei einer solchen einstückigen Ausbildung von dem Befestigungsabschnitt und einem Kontaktabschnitt liegt natürlich auch am Befestigungsabschnitt Spannung an. Dies ist aber nicht von Nachteil, da der Schiebekörper 13 üblicherweise aus isolierendem Material besteht und auch die Befestigungseinrichtung 31 ohne weiteres nach außen gut isolierend ausgebildet sein kann.

## Patentansprüche

1. Urologisches Resektoskop (1) beinhaltend eine HF-beaufschlagbare Elektrode (18) mit einem Elektrodenträger (19, 119, 219), der zumindest einen außen isolierten Leiterdraht (20, 120) aufweist, und ein axial erstrecktes Schaftrohr (2), das mit seinem proximalen Ende an einem Hauptkörper (3) befestigt ist, wobei proximal von diesem, gegenüber dem Hauptkörper achsparallel verschiebbar ein Schiebekörper (13) gelagert ist, der eine Aufnahme (24) aufweist, in die der proximale Endbereich des in Montagestellung im Schaftrohr (2) axial verschiebbar gelagerten, den Hauptkörper (3) durchlaufenden Elektrodenträgers (19, 119, 219) einsteckbar ist zur Befestigung eines Befestigungsabschnittes (25) des Elektrodenträgers (19, 119, 219) mit einer Befestigungseinrichtung (31) des Schiebekörpers (13) und zur Kontaktierung eines ersten Kontaktabschnittes (26, 126) des Elektrodenträgers (19, 119, 219) mit einer ersten Kontaktiereinrichtung (28, 29, 128, 129) des Hauptkörpers (3), **dadurch gekennzeichnet, daß** der Befestigungsabschnitt (25) proximal vom ersten Kontaktabschnitt (26, 126) angeordnet ist.

2. Urologisches Resektoskop nach Anspruch 1, **dadurch gekennzeichnet, daß** der Elektrodenträger (119, 219) zwei Leiterdrähte (20, 120) mit zwei zugeordneten Kontaktabschnitten (26, 126) aufweist.

3. Urologisches Resektoskop nach Anspruch 2, **dadurch gekennzeichnet, daß** der Befestigungsabschnitt (25) proximal von beiden Kontaktabschnitten (26, 126) angeordnet ist.

4. Urologisches Resektoskop nach Anspruch 1 oder 2, **dadurch gekennzeichnet, daß** der Befestigungsabschnitt (25) abweichend von der übrigen Querschnittsausbildung des Elektrodenträgers (19, 119, 219) ausgebildet ist.

5. Urologisches Resektoskop nach Anspruch 1 oder 2, **dadurch gekennzeichnet, daß** der Befestigungsabschnitt (25) massiv aus Metall ausgebildet ist.

6. Urologisches Resektoskop nach Anspruch 1 oder 2, **dadurch gekennzeichnet, daß** der Befestigungsabschnitt (25) und wenigstens ein Teil eines benachbarten Kontaktabschnittes (26, 126) einstückig ausgebildet sind.

7. Urologisches Resektoskop nach Anspruch 2, **dadurch gekennzeichnet, daß** der Befestigungsabschnitt (25) zwischen den Kontaktabschnitten (26, 126) liegt und aus isolierendem Material hergestellt ist.

## Claims

1. A urological resectoscope (1) including an electrode (18), to which high frequency may be applied, with an electrode carrier (19, 119, 219), which includes at least one externally insulated conductive wire (20, 120), and an axially extending tubular shaft, the proximal end of which is connected to a main body (3), mounted proximally of which is a sliding body (13), which is movable parallel to the axis with respect to the main body and has a receptacle (24), into which the proximal end region of the electrode carrier (19, 119, 219), which is axially movably mounted in the tubular shaft (2) in the installed position and extends through the main body (3), may be inserted to connect a connecting section (25) of the electrode carrier (19, 119, 219) to a connecting device (31) on the sliding body (13) and to electrically connect a first contact section (26, 126) on the electrode carrier (19, 119, 219) to a first contact device (28, 29, 128, 129) on the main body (3), **characterised in that** the connecting section (25) is arranged proximally of the first contact section (26, 126).

2. A urological resectoscope as claimed in Claim 1, **characterised in that** the electrode carrier (119, 219) includes two conductive wires (20, 120) with two associated contact sections (26, 126).

3. A urological resectoscope as claimed in Claim 2, **characterised in that** the connecting section (25) is arranged proximally of the two contact sections (26, 126).

4. A urological resectoscope as claimed in Claim 1 or 2, **characterised in that** the connecting section (25) has a cross-sectional shape differing from that of the remainder of the electrode carrier (19, 119, 219).

5. A urological resectoscope as claimed in Claim 1 or 2, **characterised in that** the connecting section (25) is of massive, metallic construction.

6. A urological resectoscope as claimed in Claim 1 or 2, **characterised in that** the connecting section (25) and at least a portion of an adjacent contact section (26, 126) are of integral construction.

7. A urological resectoscope as claimed in Claim 2, **characterised in that** the connecting section (25) is situated between the contact sections (26, 126) and is made of insulating material.

## Revendications

1. Résectoscope urologique (1) comportant une électrode (18) à laquelle peut être appliqué un courant HF associée à un porte-électrode (19, 119, 219) présentant au moins un fil conducteur (20, 120) isolé à l'extérieur, et une gaine (2) s'étendant dans le sens axial fixée par son extrémité proximale à un corps principal (3) du côté proximal duquel est logé un corps coulissant (13) lequel peut coulisser par rapport au corps principal parallèlement à son axe et présente un logement (24) dans lequel la section d'extrémité proximale du porte-électrode (19, 119, 219) logé de façon à pouvoir, en position de montage dans la gaine (2), coulisser axialement et traversant le corps principal (3) peut être enfichée en vue de la fixation d'une section de fixation (25) du porte-électrode (19, 119, 219) à l'aide d'un dispositif de fixation (31) du corps coulissant (13) et du contactage d'une première section de contactage (26, 126) du porte-électrode (19, 119, 219) avec un premier dispositif de contactage (28, 29, 128, 129) du corps principal (3), **caractérisé en ce que** la section de fixation (25) est agencée en une position proximale par rapport à la première section de contactage (26, 126).

2. Résectoscope urologique selon la revendication 1, **caractérisé en ce que** le porte-électrode (119, 219) présente deux fils conducteurs (20, 120) avec deux sections de contactage (26, 126) correspondantes.

3. Résectoscope urologique selon la revendication 2, **caractérisé en ce que** la section de fixation (25) est placée du côté proximal des deux sections de contactage (26, 126).

4. Résectoscope urologique selon la revendication 1 ou 2, **caractérisé en ce que** la conformation de la section de fixation (25) est différente de la forme de la coupe transversale du porte-électrode (19, 119, 219).

5. Résectoscope urologique selon la revendication 1 ou 2, **caractérisé en ce que** la section de fixation (25) est réalisée en métal de conformation massive.

6. Résectoscope urologique selon la revendication 1 ou 2, **caractérisé en ce que** la section de fixation (25) et une partie au moins d'une sections de contactage (26, 126) voisine sont réalisées d'un seul tenant

7. Résectoscope urologique selon la revendication 2, **caractérisé en ce que** la section de fixation (25) est située entre les sections de contactage (26, 126) et est réalisée en un matériau isolant.
